Europäisches Patentamt

**European Patent Office**

**Office européen des brevets**

(19)

(11) Publication number: **0 129 607**
**A1**

(12)

# EUROPEAN PATENT APPLICATION

published in accordance with Art. 158(3) EPC

(21) Application number: **84900302.5**

(22) Date of filing: **27.12.83**

Data of the international application taken as a basis:

(86) International application number:
**PCT/JP83/00459**

(87) International publication number:
**WO84/02461 (05.07.84 84/16)**

(51) Int. Cl.⁴: **A 61 B 17/41**
**A 45 D 26/00**

(30) Priority: **28.12.82 JP 195907/82 U**
**14.07.83 JP 126870/83**

(43) Date of publication of application:
**02.01.85 Bulletin 85/1**

(84) Designated Contracting States:
**DE FR GB**

(71) Applicant: **YA-MAN LTD**
**6 Floor Nisshin Bldg. 9-5 Nihonbashikayabacho 2-chome**
**Chuo-ku Tokyo 103(JP)**

(72) Inventor: **YAMAZAKI, Iwao**
**2 Floor Yamaya Bldg. 3-9, Monzennakacho 2-chome**
**Koto-ku Tokyo 135(JP)**

(72) Inventor: **NAKAMICHI, Yuji**
**2 Floor Yamaya Bldg. 3-9, Monzennakacho 2-chome**
**Koto-ku Tokyo 135(JP)**

(72) Inventor: **ABE, Keizo**
**2 Floor Yamaya Bldg. 3-9, Monzennakacho 2-chome**
**Koto-ku Tokyo 135(JP)**

(72) Inventor: **OKUDERA, Tatsuya**
**2 Floor Yamaya Bldg. 3-9, Monzennakacho 2-chome**
**Koto-ku Tokyo 135(JP)**

(74) Representative: **Säger, Manfred, Dipl.-Ing. et al,**
**Patentanwälte Dipl.-Ing. Otto Flügel Dipl.-Ing. Manfred**
**Säger Cosimastrasse 81**
**D-8000 München 81(DE)**

(54) **SYSTEM FOR AUTOMATING BEAUTY TREATMENT.**

(57) A system for automating devices and tools for beauty treatments such as permanent depilation, facial treatment, skin treatment, weight-reduction, and so forth, with measuring devices (1,2,3,4,5,6,7) for measuring various conditions of the part of the body being subjected to the beauty treatments, e.g., skin moisture, temperature, pH, etc. Outputs obtained from these measuring devices are arithmetically processed by operational control sections (11,12,13,14) so that the operational conditions of beauty treatment devices (22,23,24,25), e.g., the output intensities and the on/off operations of the devices, can be controlled by the results of such arithmetic operations.

FIG. 1

SPECIFICATION


AUTOMATIC SYSTEM FOR BEAUTY TREATMENTS


Technical Field

This invention relates to an automatic system for beauty treatment that decides the operating conditions of various beauty equipments. It activates metabolism through stimulation of skin by applying an electromagnetic wave of an infrared ray or laser. This system includes an eternal epilator to remove unwanted hair by applying, either separately or in combination, direct current voltage, high frequency output or laser beam.


Background Technique

When performing eternal epilation or other beauty treatment, the changing or setting up of various conditions is required, depending on the condition of the hair or skin to be treated. Previously, these conditions have been decided according to the treatment technician's experience or intuition. However, there, these conditions are very complicated and need full-scale measuring equipment or an analyzer based on physical or chemical principles. Therefore, the setting up of suitable conditions require great skill. Especially when precise judgment has to be made on conditions based on more than two data that are obtained by this equipment, extremely high level experience or intuition is required. Accordingly, high level skill is required of the treatment technicians. Technicians with little experience have great difficulty in setting up conditions quickly.

Also, it is not efficient to spend hours obtaining data by measurement or interview for each eternal epilation or other beauty treatment. When performing a special treatment, a few measurement results and data from a couple of past measurements are sufficient as information sources. Yet, treatment technicians spend lots of time considering and comparing the data in the past or the data from other common cases.

## Disclosure of the Invention

It is the principal of the present invention to provide an automatic system for beauty treatments including eternal epilation, characterized by possessing the following: data measuring apparatuses that measure moisture, temperature, pH, etc. of the area to be treated and an operation control unit which sets and provides the operating conditions needed for eternal epilator and other various beauty equipments based on various data from the above data measuring equipment. This automatic system for beauty treatments makes it possible to control various output apparatuses under the best treatment conditions. The best conditions are obtained through a general analysis of the data obtained from an interview and data such as moisture, temperature, pH, skin color and elasticity, form, oil content of the skin of the area to be treated. As a result, a desirable beauty treatment under the best conditions is provided at any time without high-level expertise or intuition.

Brief Description of the Drawings

Fig. 1 is a block diagram that illustrates the basics of the automatic beauty treatment system of this invention including eternal epilation.

Fig. 2 is a basic flow chart of the pH measuring apparatus in Fig. 1.

Fig. 3 is a block diagram that illustrates the outline of the computer and memory part of Fig. 1.

Fig. 4 is a flow chart that illustrates the basic system of eternal epilator of Fig. 1.

Fig. 5 is a selection flow chart of a hair style that is a part of the entire body beauty treatment of Fig. 1.

Fig. 6 is a block diagram that illustrates a working example of an automatic system for beauty treatment of this

- 3 -

invention as applied to the eternal epilator.

## Best Form for Working of the Invention

This invention will hereinafter be disclosed, referring to the attached illustration of working example.

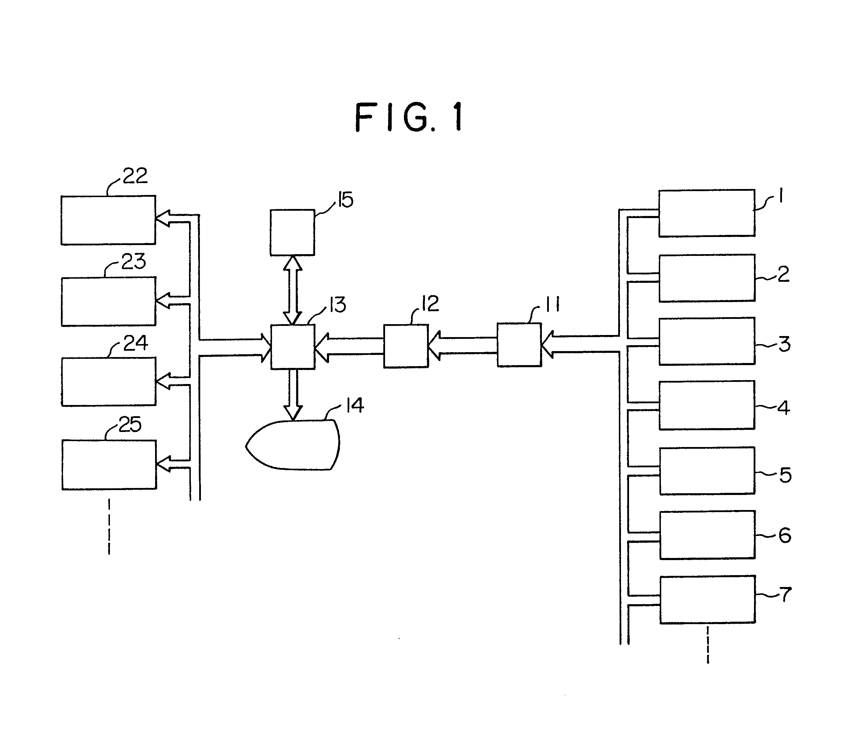

Fig. 1 illustrates construction of automatic system of this invention which consists of measuring apparatuses shown in reference marks (1) to (7), operation control unit shown in reference marks (11) to (14) and various beauty equipments shown in reference marks (22) to (25).

Various measuing apparatuses are used as measuring apparatuses (1) to (7) as the occasion calls. In the case of eternal epilator, the objects are moisture, pH, temperature, color, elasticity of the skin and thickness of hair, etc. In compliance with these conditions, the following must be considered: whether the output of eternal epilator should be either direct current or high frequency, or both; whether they should be applied continuously or applied in a pulse-like manner; and what kind of output, i.e. big, medium, small, etc. should we use. Following is a description of the relationship between these conditions and eternal epilation or other beauty treatments.

First, the quantity of skin moisture effects the whole process and each treatment speed remarkably changes according to its quantity. Especially in eternal epilation, the quantity of skin moisture is so important that the

- 4 -

relationship between eternal epilation and the quantity of skin moisture should be mentioned.

At present, general eternal epilation treatment is done by electricity.  Existing epilation methods are an electrolysis method using direct current, a high-frequency method and a combination thereof.  In an eternal epilation with electrolysis, direct current-voltage is applied to an anode in contact with skin, for example grasped in hand, and a cathod put in follicle to attack a follicle by alkalinity caused through electrolysis by the moisture lying the follicle, which results in an eternal epilation.

An eternal epilation done by high-frequency heats moisture in a follicle through dielectric heating using electrodes similar to those mentioned above.

The combined type eternal epilation is performed through synergistic effect by using the above combined equipment.

The quantity of the skin moisture, especially in hair follicles, greatly influences the epilation results. However, it is impossible to perceive the quantity of the moisture of skin, especially in these follicles by just looking or touching by hand.  Therefore, the operation has been done depending on an operator's intuition up to now.  It is impossible to perform suitable epilation on the basis of the exact

- 5 -

quantity of moisture in follicle because it differs by age, sex and the portion. Thus, we have been having problems of overtreatment or insufficient treatment.

Also the range of the value of pH of normal skin is from 4.5 to 6.5, thus acidic. There is a considerable big difference depending upon individuals and this value varies even in a same person due to sweating or other conditions. The value of pH greatly influences each beauty treatment condition.

Furthermore, the skin temperature is important since an infrared ray irradiation, etc. are included in the weight reduction of the entire body. It is recognized that what we call an acupuncture spot has a lower temperature than the other parts of the body and it is important because it helps to detect the parts to which laser irradiation is to be applied.

It is well known that cosmetics should be selected depending on the color of the skin, i.e. pale or suntanned. The color of the skin also greatly influences eternal epilation and other beauty treatments. Up to the present, the color of the skin has been judged inaccurately by the naked eye because of the background color or illumination.

The skin elasticity is an important factor of the beauty treatment since it shows the degree of the elasticity.

As a matter of fact, the characteristic of the hair taken as a sample should be understood thoroughly and used as data because the shape and weight of the hair treated is an important factor in the treatment.

This embodiment is equipped with the moisture measuring apparatus (1), pH measuring apparatus (2), temperature measuring apparatus (3), color measuring apparatus (4), pressure measuing apparatus (5), form measuring apparatus (6) and weight measuring apparatus (7). It is understood that other systems can be constructed as needed.

Common equipment by which an electric output is obtained based on the principle of physics or chemistry is useful for each of the above measuring apparatus. The following is a disclosure of the most general construction.

Moisture measuring apparatus (1) measures, for example, the moisture through the electrical resistance between the two electrodes that touch the skin. Also a humidity sensor is useful.

The well known glass electrode can be used as the pH measuring apparatus (2). The glass electrode is suitable for the pH measurement of the surface of the skin. In the case that the pH measurement of the hair follicle is needed, the pH indicator can be used at the root of the treated hair.

The pH measurement by the glass electrode has several advantages. For example, the restriction of the object

- 7 -

measured is small, continuous measurement is possible and so on, and it is adopted in the Japanese Industrial Standard.

The thermoelectric couple or thermistor, which measures electromotive force or electric resistance through contact with Device Under Test, is useful as the temperature measuring apparatus (3). Also, the measurement using infrared rays in non-contact may be adopted.

The color sensor, which has a color filter added to a photo diode, is with ease used as the color measuring apparatus (4). For example, a display is easily done by XYZ color display system (CIE*1931*XYZ color display system) or L'a'b' color display system (CIE1976*L'a'b'* - Color Space) through the amorphous integrated circuit type color sensor or monolithic color sensor.

A pressure sensor of electrostatic capacitance type or other types is useful as the pressure measuring apparatus (5).

The CCD or other image sensors can be used as the form measuring apparatus (6). The digital type micrometer or slide calipers can be used, but they are not suitable for the measurement of extremely minute hairs.

A chemical balance can be used as the weight measuring apparatus (7) and the measuring value of hair should be inputted. Also, sensors that measure minute weight or mass can be used.

- 8 -

The operation control part consists of A/C converter (11), interface (12), computer (13) and display part (14). The parts may comprise well-known elements. It is preferable that the display part (14) is constructed to be able to display switching of the detected value of each measuring part, the result of operation and the operating condition of each beauty equipment which will be mentioned later.

The beauty equipment consists of beauty equipment for the entire body (22), facil treatment (23), weight reduction equipment (24) and eternal epilator (25). Each unit employs CPU that obtains the best control with A/D converter and D/A converter suitable for transferring the signal to and from the operation control unit.

Each beauty equipment and the main data needed by each equipment will hereinafter be disclosed. First, the moisture amount, temperature, pH and the hair condition (weight) are important in an eternal epilator. Moreover, the skin collor, skin elasticity and so on are important to be compared with the previous measuring value.

In total body beauty, the moisture amount, temperature, facial form and physique are important. In a weight reduction, it is important to understand how weight, moisture amount, temperature, pH or facial form, and physique change. These data can be measured by a simple measuring apparatus employed in each beauty equipment and can be transmitted to the computer as occasion demands.

- 9 -

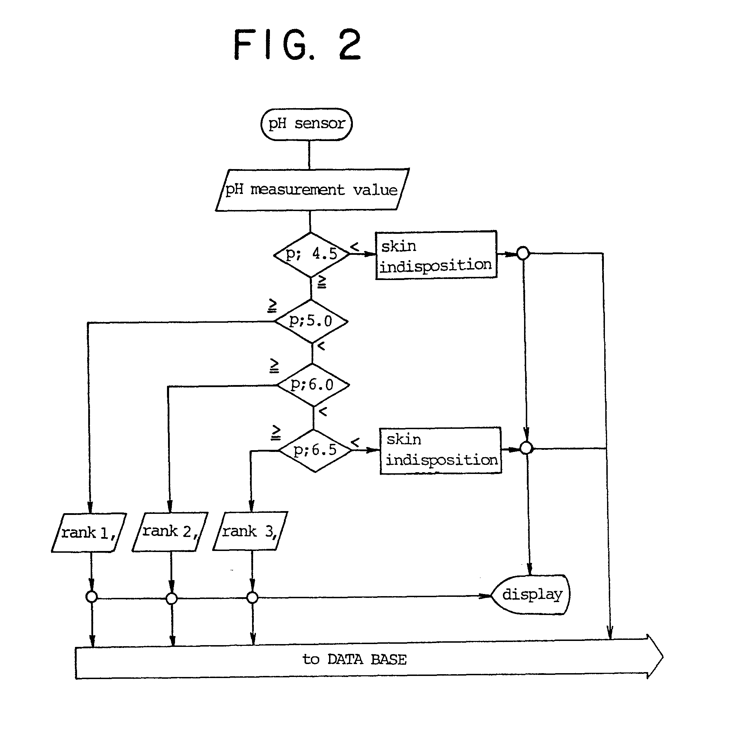

Fig. 2 shows a flow chart of the pH measuring apparatus (2). It is possible to indicate warning on the display unit when the pH value is either less than 4.5 or more than 6.5. In this figure, the pH value between 4.5 and 6.5 is divided into three sections. However, the number of section may properly be increased if necessary.

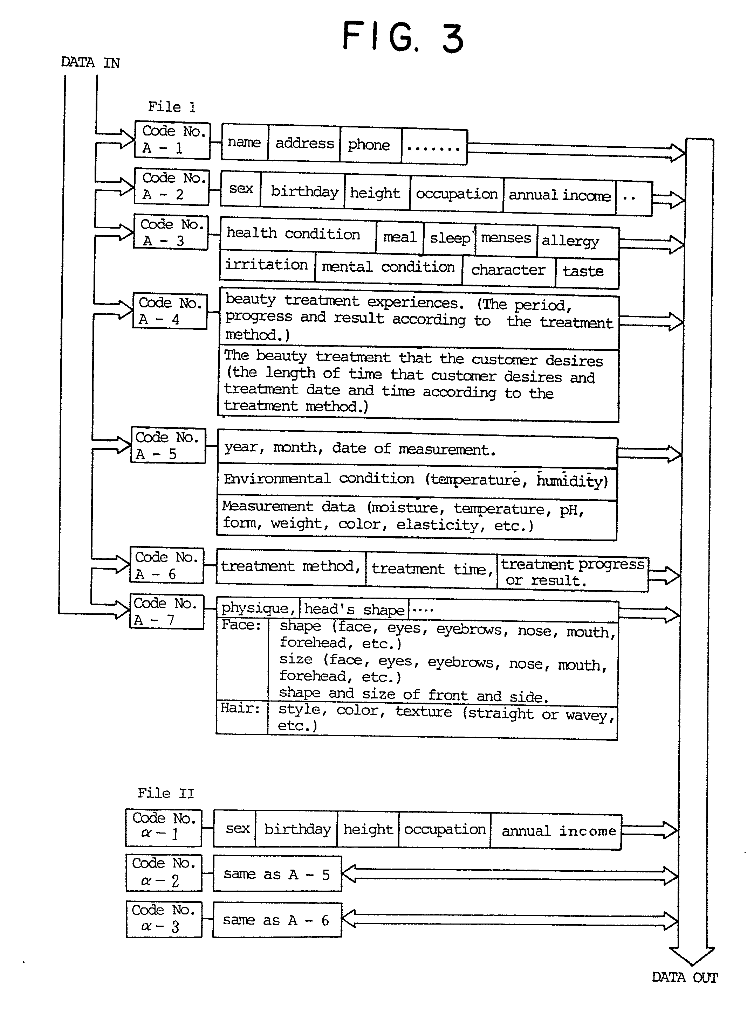

Fig. 3 is a block diagram that shows the outline of the computer (13) and the memory storage part (15). Out of the code numbers used here, cord No. A is a part that is common in File I and cord No. of another person is expressed in B. Therefore, the File I is regarded as the individual card.

A - 1 shows the inherent data for the code No. A.

A - 2 shows data that are almost unnecessary to be changed once they are input.

A - 3 shows data to be filed after every measurement is performed.

A - 4 shows the basic data obtained through an interview, etc.

A - 5 shows the measuring data that are a basis for beauty treatment.

A - 6 shows data obtained through treatment performance.

A - 7 shows data that mainly relate to display.

An alpha part is a code that is common in File II and the data base part primarily for statistic process

- 10 -

and the value processed there are used to be compared with other data.

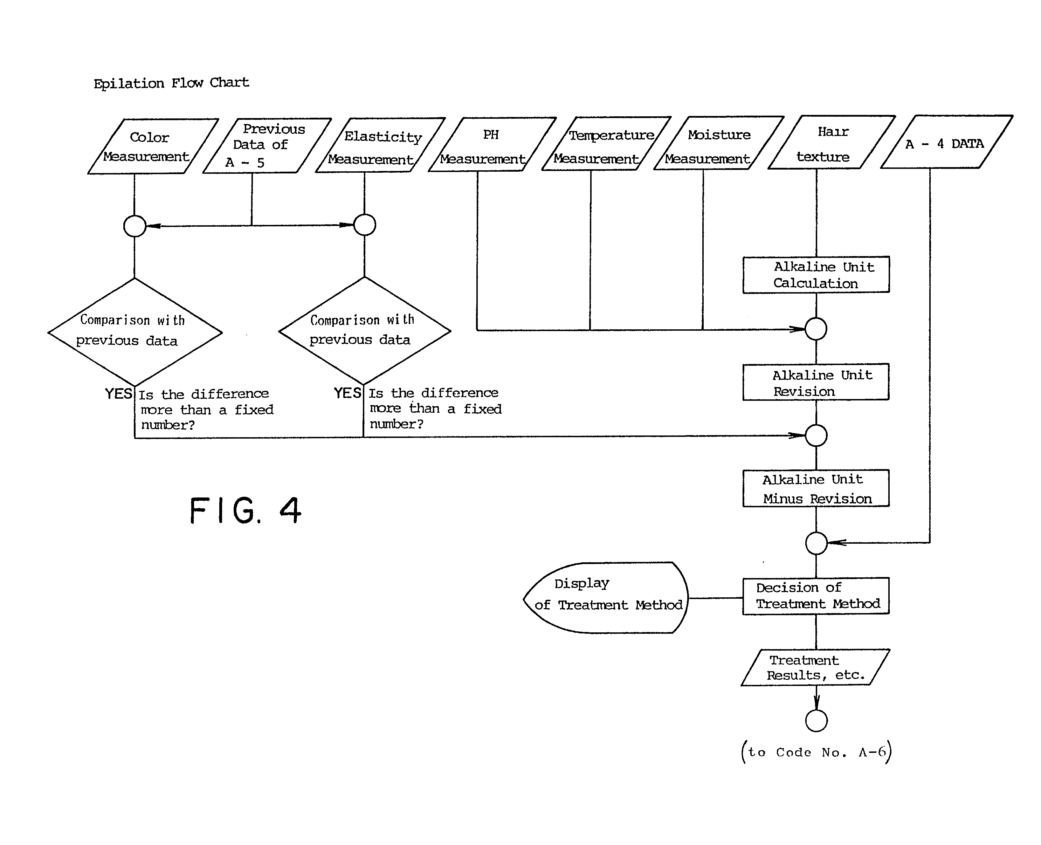

Fig. 4 is a flow chart that shows the basic construction of the eternal epilator. In this figure, the alkali unit number, which is a measure of eternal epilation treatment, is calculated through the form of hair such as thickness or length. The alkali unit number is modified in consideration of factors obtained through each sensor of pH, temperature and mosture.

Conserning the color of skin, the upper limit is set for the alkali unit or the unit number is subtracted, for example, in case where, it has drastically changed, compared with the previous measurement, because of suntan, etc., or skin elasticity has drastically changed. The result of these treatments are filed in Code No. A - 6 together with the data of the method and process.

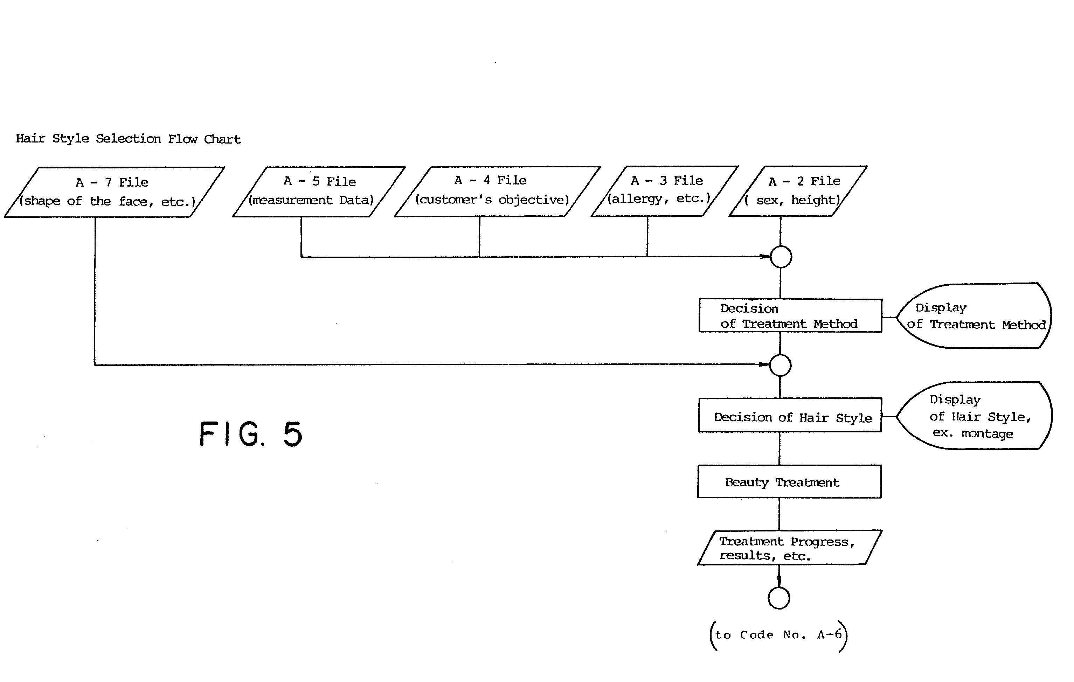

Fig. 5 is a flow chart to select a hair style that is appropriate for total body beauty. It is also possible to select a suitable hair style treatment (e.g. permanent wave), how to make-up, suitable cosmetics, etc.

In this automatic system according to this invention, the best conditions are calculated by the operation according to the conditions obtained through each kind of the measuring apparatuses (1) to (7) previously mentioned. The beauty equipments (22) to (25) are most suitably controlled by the results of the above operation. Accordingly, it is possible to perform a safe, comfort and secure beauty treatment under the most suitable conditions at all times, regardless of the operating technician's experience or intuition.

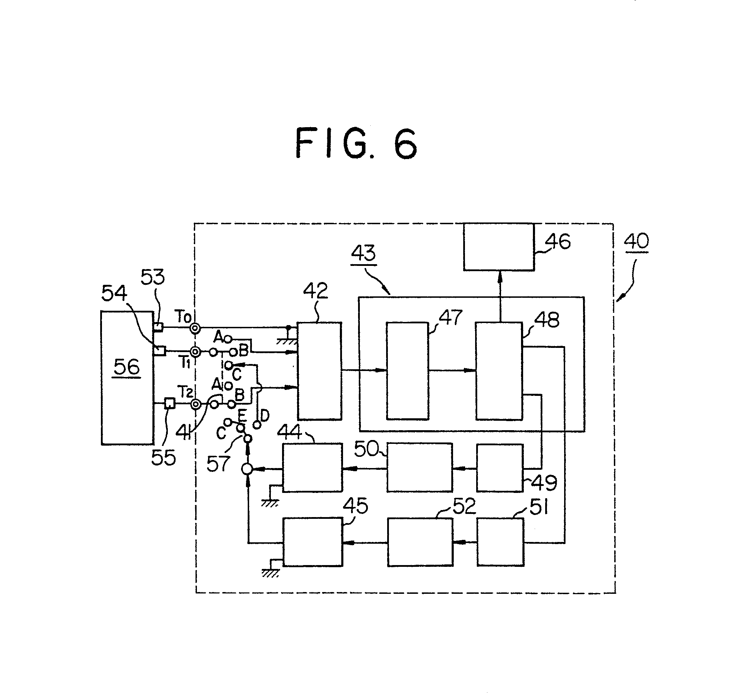

Fig. 6 specifically shows a block diagram of an eternal epilator, a part of the automatic system for beauty treatment according to this invention, and indicates the construction which omits the part of power supply of the equipment. The eternal epilator shown by the broken line (40) possesses the following: changeover switch (41), detector (42), central processing operation unit (CPU) (43), high frequency generator (44), direct current generator (45), memory storage parts (49), (51), which control (44) and (45) according to the output of CPU (43). It also possesses control units (50) and (52) and also possesses the displayer (46) which indicates the measurement value, if necessary. The CPU (43) consists of the input processing unit (47) and operation unit (48). Moreover, an eternal epilator (40) is equipped with terminals for input and output, $T_0$, $T_1$ and $T_2$.

The terminal $T_0$ connects with a metal hand bar (53) as an electrode, which is used in common in the moisture measurement and eternal epilation treatment. The terminal $T_1$ connects with electrode (54) used for measuring skin moisture or pre-and post-treatment of epilation. Terminal $T_2$ connected with a probe electrode (55) used for measuring moisture in follice and for epilation. Electrodes (53), (54) and (55) are contacted with and plugged in a human body (56) that needs epilation.

The following explains the operation of the eternal epilation according to this invention. It becomes possible to measure skin moisture and perform pre-and post-treatment of epilation where the changeover switch (41) contacts with a point of contact A. And it becomes possible to measure moisture in the hair follicle when the changeover switch contacts with a point of contact B. An epilation is performed under the condition that the changeover switch contacts with a point of contact C.

When measuring the skin moisture, an electrode (54) contacts with a part of skin to be measured after the metal hand electrode (53) is held by a customer in his or her hand setting the changeover switch (41) to the point of contact A. After measuring the above under this condition, the detector (42) detects input from the metal hand electrode (53) and the electrode (54) and then transfers the detected input to an input processing unit (47) in the CPU (43). The detected value is processed in the operation unit (48) and displayed on the displayer (46), if necessary.

In case of measuring moisture in hair follicle, the probe electrode (55) should be plugged into a follicle to be epilated after a metal hand electrode (53) is held by a customer in his or her hand setting the changeover switch (41) to the point of contact B. After measuring the above under this condition, a detector (42) detects an electric resistance between the metal hand electrode (53) and the probe electrode (55) and the detected value is processed in the same manner as in the above and then displayed on the displayer (46).

During the epilation treatment, the most suitable voltage and application time are set based on a result of each moisture measurement of the above. These controls are done through the operation unit (48) in the CPU (43) and a control operation is done by the control units (50) and (52) through data stored in the first and second storage units (49) and (51). The output of the control unit (50) controls the direct current generator (44) and the output of the control unit (52) controls the high frequency generator (45). As a result, the epilation treatment is performed under the most suitable conditions for the status of moisture in follicle and of skin.

A pre-(and post-) treatment of an eternal epilation is done by switching an addition switch (57) to a point of contact D, setting the changeover switch (41) to the point of contact C and applying a weak voltage between the metal hand electrode (53) and the electrode (54). By performing the pre-treatment, the skin pores are opened, and plugging of the epilation probe electrode is facilitated to thereby ensure a reliable epilation. The after-treatment of epilation is done by tightening pores through reversing the polarity of two electrodes and neutralizing an entire part.

After the pre-treatment of epilation, the point of contact of an addition switch (57) should be set to E side while leaving the changeover switch (41) as it is (the point of contact C). The epilation treatment is performed with a combination of time and voltage set based on the previous measurement and of direct current and high frequency after inserting the tip of the probe electrode (55) in pores. These setting values are decided according to the moisture measurement value obtained immediately before the epilation treatment under the most suitable condition for age, sex, follicle and skin and so on. Therefore, it makes it possible to perform an appropriate epilation treatment without requiring the very high level of skill since an operator's intuition is not needed.

It would be evident that much transformation and modification can be done within the range of this invention based on this disclosure and working examples of this invention.

- 15 -

WHAT IS CLAIMED IS: .

1. An automatic system for deciding operating conditions of an eternal epilator that removes unwanted hair by applying an electromagnetic wave such as direct current voltage, low frequency, high frequency, infrared ray, ultra-violet ray and laser individually and/or combining the above, or of various beauty equipment that activates metabolism through stimulation of skin by applying the above, characterized in that said automatic system for beauty treatment includes an eternal epilation comprising data measuring apparatuses to measure moisture, temperature, pH and so on of an area to be treated and an operation control unit which sets and provides operating conditions needed for said various beauty equipments based on various data from said data measuring apparatuses.

2. The automatic system as described in Claim 1, wherein the system comprises an eternal epilator that operates by either or both of the operating conditions set individually or through the above automatic system.

3. The automatic system as described in Claim 2, wherein the said eternal epilator performs epilation by applying either or both of direct current voltage or high frequency output to follicle and also includes the moisture amount measurement system consisting of an electrode and a detector that measure the moisture in skin or follicle as an electric signal, and a central processing unit (CPU) that processes the output of said detector in operation and then generates display output and output for control.

4. The automatic system as described in Claim 1, wherein the system includes weight reduction and facial treatment equipments that operate under the operating condition set by either or both of individually or through the above automatic system.

5. The automatic system as described in Claim 2, wherein the system includes weight reduction and facial treatment equipments.

6. The automatic system as described in Claim 5, wherein the automatic system includes information processing system that processes information obtained through each measuring apparatus.

7. The automatic system as described in Claim 6, wherein the automatic system includes customer management system that processes customer information.

# FIG. 1

0129607

0129607

# FIG. 2

```
        ( pH sensor )
              |
   /pH measurement value/
              |
          < p; 4.5 >  <── ┌──────────────┐ ──> ○ ─────────┐
              | ≧        │    skin      │                 │
              |          │ indisposition│                 │
   ≧ ┌──── < p;5.0 >     └──────────────┘                 │
     │          | <                                       │
     │  ≧ ┌── < p;6.0 >                                   │
     │    │      | <                                      │
     │    │  ≧ ┌ < p;6.5 > <── ┌──────────────┐ ──> ○ ──┐ │
     │    │    │         │    skin      │         │ │
     │    │    │         │ indisposition│         │ │
     │    │    │         └──────────────┘         │ │
     │    │    │                                  │ │
  /rank 1,/ /rank 2,/ /rank 3,/                   │ │
     │      │      │                              │ │
     ○      ○      ○ ──────────> ( display )      │ │
     │      │      │                              │ │
     ▼      ▼      ▼                              ▼ ▼
   ┌─────────────────── to DATA BASE ──────────────────>
```

# FIG. 3

0129607

DATA IN

**File 1**

| Code No.<br>A − 1 | name | address | phone | ....... |

| Code No.<br>A − 2 | sex | birthday | height | occupation | annual income | .. |

| Code No.<br>A − 3 | health condition | meal | sleep | menses | allergy |
| | irritation | mental condition | character | taste |

| Code No.<br>A − 4 | beauty treatment experiences. (The period, progress and result according to the treatment method.) |
| | The beauty treatment that the customer desires (the length of time that customer desires and treatment date and time according to the treatment method.) |

| Code No.<br>A − 5 | year, month, date of measurement. |
| | Environmental condition (temperature, humidity) |
| | Measurement data (moisture, temperature, pH, form, weight, color, elasticity, etc.) |

| Code No.<br>A − 6 | treatment method, | treatment time, | treatment progress or result. |

| Code No.<br>A − 7 | physique, | head's shape | .... |
| | Face: | shape (face, eyes, eyebrows, nose, mouth, forehead, etc.)<br>size (face, eyes, eyebrows, nose, mouth, forehead, etc.)<br>shape and size of front and side. |
| | Hair: | style, color, texture (straight or wavey, etc.) |

**File II**

| Code No.<br>α − 1 | sex | birthday | height | occupation | annual income |

| Code No.<br>α − 2 | same as A − 5 |

| Code No.<br>α − 3 | same as A − 6 |

DATA OUT

Epilation Flow Chart

FIG. 4

(to Code No. A-6)

Hair Style Selection Flow Chart

FIG. 5

A - 7 File
(shape of the face, etc.)

A - 5 File
(measurement Data)

A - 4 File
(customer's objective)

A - 3 File
(allergy, etc.)

A - 2 File
( sex, height)

Decision
of Treatment Method

Display
of Treatment Method

Decision of Hair Style

Display
of Hair Style,
ex. montage

Beauty Treatment

Treatment Progress,
results, etc.

(to Code No. A-6)

# FIG. 6

# INTERNATIONAL SEARCH REPORT

0129607

International Application No. PCT/JP83/00459

**I. CLASSIFICATION OF SUBJECT MATTER** (if several classification symbols apply, indicate all) ³

According to International Patent Classification (IPC) or to both National Classification and IPC

Int. Cl³   A61B 17/41, A45D 26/00

**II. FIELDS SEARCHED**

| Minimum Documentation Searched ⁴ |
|---|

| Classification System | Classification Symbols |
|---|---|
| IPC | A61B 17/41, A45D 26/00 |

| Documentation Searched other than Minimum Documentation<br>to the Extent that such Documents are Included in the Fields Searched ⁵ |
|---|
| Jitsuyo Shinan Koho          1960 - 1983<br>Kokai Jitsuyo Shinan Koho    1971 - 1983 |

**III. DOCUMENTS CONSIDERED TO BE RELEVANT ¹⁴**

| Category* | Citation of Document, ¹⁶ with indication, where appropriate, of the relevant passages ¹⁷ | Relevant to Claim No. ¹⁸ |
|---|---|---|
| A | JP, A, 55-76641 (Inbanes International Corp.), 9 June 1980 (09. 06. 80)<br>Page 4, lower right column, line 10 to page 6, upper right column, line 15 & DE, A1, 2,943,847 & FR, A1, 2,442,623 & GB, A, 2,039,053 & US, A, 4,216,775 & US, A, 4,295,467 & CA, A1, 1,142,599 | 1 |

* Special categories of cited documents: ¹⁵

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search ² | Date of Mailing of this International Search Report ² |
|---|---|
| March 13, 1984   (13. 03. 84) | March 19, 1984   (19. 03. 84) |

| International Searching Authority ¹ | Signature of Authorized Officer ²⁰ |
|---|---|
| Japanese Patent Office | |

Form PCT/ISA/210 (second sheet) (October 1981)